# EUROPEAN PATENT SPECIFICATION

(11) **EP 2 129 384 B1**
(45) Date of publication and mention of the grant of the patent: **13.04.2016**
(21) Application number: 08737299.1
(22) Date of filing: 07.03.2008
(51) Int. Cl.: A61K 35/28, A61K 35/32, A61L 27/38

(54) **COMPOSITION CONTAINING A MEDULLARY CONCENTRATE SUPPORTED BY A SCAFFOLD**
ZUSAMMENSETZUNG ENTHALTEND EIN MEDULLÄRES KONZENTRAT AUF EINEM GERÜST
COMPOSÉ CONTENANT UN CONCENTRÉ MEDULLAIRE SUPPORTÉ PAR UN ÉCHAFAUDAGE

(30) Priority: 07.03.2007 IT MI20070458
(43) Date of publication of application: 09.12.2009
(73) Proprietor: Istituto Ortopedico Rizzoli, 40136 Bologna (IT)
(72) Inventor: GIANNINI, Sandro, I-55049 Viareggio (IT); BUDA, Roberto, I-40037 Sasso Marconi (IT); GRIGOLO, Brunella, I-40136 Bologna (IT)
(74) Representative: Biggi, Cristina
(86) International application number: PCT/IB2008/000530
(87) International publication number: WO 2008/107785

(56) References cited:
- WO-A-02/070023
- CHANG ET AL: "P207 Repair of large full-thickness articular cartilage defects by transplantation of autologous uncultured bone-marrow-derived mononuclear cells" 1 January 2007 (2007-01-01), OSTEOARTHRITIS AND CARTILAGE, BAILLIERE TINDALL, LONDON, GB, PAGE(S) B140 , XP022221725 ISSN: 1063-4584 abstract
- GANGJI VALÉRIE ET AL: "TREATMENT OF OSTEONECROSIS OF THE FEMORAL HEAD WITH IMPLANTATION OF AUTOLOGOUS BONE-MARROW CELLS. A PILOT STUDY" 1 June 2004 (2004-06-01), JOURNAL OF BONE AND JOINT SURGERY, JOURNAL OF BONE AND JOINT SURGERY. BOSTON, US, PAGE(S) 1153 - 1160 , XP009085962 ISSN: 0021-9355 cited in the application abstract page 1155, paragraph 3 Discussion
- XIAO Z M ET AL: "Treatment of osteonecrosis of femoral head with BMSCs-seeded bio-derived bone materials combined with rhBMP-2 in rabbits" 1 June 2008 (2008-06-01), CHINESE JOURNAL OF TRAUMATOLOGY ENGLISH EDITION,, PAGE(S) 165 - 170 , XP022706557 ISSN: 1008-1275 [retrieved on 2008-06-01] abstract Methods Discussion
- CHANG FEI ET AL: "Repair of large full-thickness articular cartilage defects by transplantation of autologous uncultured bone-marrow-derived mononuclear cells." JOURNAL OF ORTHOPAEDIC RESEARCH : OFFICIAL PUBLICATION OF THE ORTHOPAEDIC RESEARCH SOCIETY JAN 2008, vol. 26, no. 1, January 2008 (2008-01), pages 18-26, XP008105010 ISSN: 1554-527X
- GIANNINI SANDRO ET AL: "Histological and immunohistochemical analysis of an allogenic bone graft engineered with autologous bone marrow mononuclear cells in the treatment of a large segmental defect of the ulna. A case report." LA CHIRURGIA DEGLI ORGANI DI MOVIMENTO APR 2008, vol. 91, no. 3, April 2008 (2008-04), pages 171-175, XP008105015 ISSN: 0009-4749
- KIM SEOK-JUNG ET AL: "Treatment of osteonecrosis of the femoral head using autologous cultured osteoblasts: a case report" 25 February 2008 (2008-02-25), JOURNAL OF MEDICAL CASE REPORTS, BIOMED CENTRAL LTD, LO, PAGE(S) 58 , XP021040524 ISSN: 1752-1947 abstract paragraphs [0003] - [0005]
- MATSUMURA ET AL: "Evaluation of tissue-engineered vascular autografts", TISSUE ENGINEERING, vol. 12, 2006, pages 3075-3083, XP002559298,
- PubMed PMIN: 16078651, one page (Rasumov et al, Patol Fiziol Eksp Ter; 2005, pp. 20-23)

## Description

### Technical Field

The present invention concerns the treatment of osteochondral articular lesions.

Purpose of the repair of the articular cartilage is to restore the integrity of the joint surface, through the regeneration of hyaline cartilage. Hyaline cartilage can be obtained by methods that employ cell transplantations of chondrocytes (or their precursors).

### Background Art

Nowadays, the state of the art involves the implant of autologous chondrocytes by arthroscopy. Such method entails a preliminary arthroscopy to harvest cartilage from the areas adjacent to the lesion or from a detached chondral fragment; the harvested chondrocytes are expanded in in vitro cell culture for a mean period of 5 weeks and seeded on biocompatible and bioabsorbable scaffolds based on hyaluronic acid, collagen or others; in a second arthroscopy the chondrocytes are implanted on scaffolds. This technique has the drawback of requiring two surgical sessions, the time for the necessary phase of in vitro cell expansion and the preparation of environments suitable for this purpose, high costs for the whole procedure, and great inconvenience for the patient. The characterizing limit of the employment of chondrocytes is the fact that they cannot be isolated if not in small quantities from cartilaginous tissue, and they require therefore a quantitative expansion in the laboratory. Such a process takes at least 15-20 days, and does not allow therefore the execution of a "one step" surgical technique.

Rasulov et al, Patol Fiziol Eksp Ter; 2005, 20-23 (in Russian), teaches usin a suspension of bone marrow-derived mesenchymal stem cells immobilized on a biodegradable membrane for treating deep burn wounds. The bone marrow-derived mesenchymal stem cells of Rasulov are purified (isolated) cells. In particular, these cells can be obtained after in lab manipulation, for example by Ficoll separation procedure and/or in vitro culturing.

Therefore, the need was particularly felt to overcome the drawbacks in terms of time, costs and inconvenience for the patient that the technique of transplantation of autologous chondrocytes involves.

### Disclosure of the Invention

The present invention refers to a composition including a medullary concentrate supported by a scaffold chosen from the group consisting of lyophilized scaffold and pre-constituted scaffold in the form of membrane or tissue.

Moreover, the present invention refers to this composition for use as a medicament, in particular, for use in the treatment of articular osteocartilaginous injuries in arthroscopic surgery in a single operating session.

A further aspect of the present invention is, a process for the preparation of a composition including a medullary concentrate supported by scaffold chosen from the group consisting of lyophilized scaffold and pre-constituted scaffold in the form of membrane or tissue of the composition, including the steps of:
preparing a concentrate of medullary aspirate by automated method of centrifugation in the operating room;
preparing a composition including the concentrate and a support scaffold chosen from the groupe consisting of lyophilized scaffold and pre-constituted scaffold in the form of membrane or tissue, said composition obtained for mixing sa id components together.

The applicants have surprisingly conceived and realized a biological material or composition including autologous mononuclear cells, more precisely, autologous mononuclear cells of medullary origin isolated from bone marrow, from peripheral blood or from fat tissue, supported by scaffold chosen from the group including lyophilized scaffold or pre-constituted scaffold in the form of membrane or tissue, with possible addition of platelet gel or other different signalling molecules. Said composition (medullary concentrate) has been conceived for the preparation of medicaments for the treatment of osteocartilaginous articular lesions to be used in arthroscopic or arthrotomic surgery, in a single operating session.

According to the present invention it is possible to perform the full treatment in a single operating session, contrarily to the methods previously used that involve two surgical sessions.

Autologous mononuclear cells, for use as a medullary concentrate according to the present invention, in fact, unlike chondrocytes, can be harvested in great quantities and packed, directly during the operating session.

In the orthopedic field its usefulness is known in the treatment of nonunions (Hemigou, et al.: J Bone Joint Surg Br 2005 Jul; 87 (7): 896-902) and necroses of the femoral head (Gangji, et al.: J Bone Joint Surg Am. 2004 Jun;86 - A (6): 1153-60). To be implanted in great number in the lesion area, such cells need a scaffold on which to be seeded.

The scaffolds that belong to the composition of the present invention, can be lyophilized scaffolds, particularly collagen of pig origin (such as Spongostan® Powder or Surgiflo®). This is a bioabsorbable and biocompatible material already broadly used in surgery to provide surgical haemostasis, which acts well as a malleable scaffold to deliver the cells to the site of the lesion, but never indicated as possible scaffold to be combined with autologous mononuclear cells, preferably those isolated from bone marrow, peripheral blood or fat.

The composition of the present invention, besides its employment in the treatment of osteocartilaginous articular injuries in a single operating session in arthroscopic or arthrotomic surgery, allows the aforementioned drawbacks of the autologous chondrocyte transplantation technique to be overcome.

One object of the present invention consists of a biological material or composition including autologous mononuclear cells supported on scaffold chosen from the group including lyophilized scaffold or pre-constituted scaffold in the form of membrane or tissue.

In a preferred form of realization of the composition of the present invention, the autologous mononuclear cells used are autologous mononuclear cells of medullary origin, particularly those harvested from the posterior iliac crest, or autologous mononuclear cells isolated from peripheral blood or from fat.

In a further even more preferred form of realization of the composition according to the invention, the scaffold supporting the autologous mononuclear cells, more precisely those of medullary origin, chosen from the group including lyophilized scaffold or pre-constituted scaffold in the form of membrane or tissue, is a biocompatible and bioabsorbable scaffold and can be constituted by various composite materials, such as natural, semi-synthetic or synthetic polymers.

The natural polymers used for the invention are selected from the group including collagen, collagen and glycosaminoglycan co-precipitates, hyaluronic acid, cellulose, polysaccharides in the form of chitin gel, chitosan, pectin or pectic acid, agar, agarose, xanthan, gellan gum, alginic acid or alginates, polymannans or polyglycans, starch and natural rubbers, and demineralized bone matrix (DBM).

The semi-synthetic polymers used for the invention are selected from the group including reticulated collagen with agents such as aldehydes or their precursors, dicarboxylic acids or their halides, diammine, derivates of cellulose, hyaluronic acid, chitin gel or chitosan, gellan gum, xanthan, pectin or pectic acid, polyglycans, polymannans, agar, agarose, natural rubber and glycosaminoglycan.

The useful synthetic polymers to achieve the invention are selected from the group including polylactic acid, polyglycolic acid or their copolymers or derivates, polydioxane, polyphosphazenes, polysulfonic resins, polyurethanes and PTFE, hydroxyapatite in paste form.

In a preferred realization of the invention the composition includes a scaffold constituted by pig collagen.

In addition, it has been found that various signalling molecules, including growth factors, can be added to the composition of the present invention to favor the multiplication and the differentiation of the autologous mononuclear cells, particularly those from packed bone marrow.

In a further preferred form of realization of the composition of the present invention, these growth factors are provided platelet gel is obtained by harvesting venous blood the day before the operation, through an automated procedure, such as Vivostat®.

The platelet gel can be placed on the surface of the biological material or composition of the present invention or on the bed of the lesion or in the composition of the paste.

The biological material or composition of the present invention can also include pharmacologically or biologically active substances. These pharmacologically and biologically active substances are selected from the group including anti-inflammatory agents, antibiotics, growth factors, and antimycotic and antiviral agents.

Another object of the present invention is the use of biological material or composition, in any of the forms described above, for the preparation of a medicament for the treatment of articular osteocartilaginous injuries in a single operating session in arthroscopic or arthrotomic surgery.

In a preferred form of realization of the present invention articular osteocartilaginous lesions are ostoarticular lesions of knees and ankles.

According to the present invention it has also been found that it is possible to reserve, in a preferred form cryopreserve, preventively the biological material or composition, according to any of the forms of preferred realization described above, to store its characteristics of cellular viability for grafts in subsequent arthroscopic or arthrotomic procedures.

Further disclosed is a method of treatment of articular osteocartilaginous lesions in a single operating session by the implant in arthroscopic or arthrotomic surgery of a biological material or composition including autologous mononuclear cells and a scaffold chosen by the group including lyophilized scaffold or pre-constituted scaffold in the form of membrane or tissue, said method including:
- Drawing of medullary blood from the posterior iliac crest during the operating session;
- Preparation of a concentrate of autologous mononuclear cells from medullary aspirate with an automated method of centrifugation in the operating room, (such as the BMAC Harvest® method or others);
- Arthroscopy or arthrotomy of the joint to be treated;
- Arthroscopic or arthrotomic cleaning of the lesion and relief of combined conditions;
- Preparation of a composition including the concentration of autologous mononuclear cells from medullary aspirate and scaffold of support chosen by the group including lyophilized scaffold or pre-constituted scaffold in the form of membrane, said composition obtained for mixing said components together;
- Implant of the composition to fill the center of infection of the osteochondral lesion in arthroscopy or arthrotomy.

In a preferred form of the method of treatment of articular osteocartilaginous injuries in a single operating session, the lyophilized scaffold is pig collagen (such as Spongostan® Powder or Surgiflo®).

In a further preferred form of realization of the method of treatment of articular osteocartilaginous injuries in a single operating session, said method also includes before the phase of drawing of the medullary blood from the iliac crest the following phases:
- Drawing of venous blood the day before the operation;
- Preparation of platelet gel with automated procedure, such as the Vivostat procedure;
- freezing of the platelet gel up to the moment of the operation;
from which the preserved platelet gel is, alternatively:
1. mixed in the composition including the concentration of autologous mononuclear cells from medullary aspirate and support scaffold; or
2. placed in the interface between the composition including the concentration of autologous mononuclear cells from medullary aspirate and support scaffold and the bed of the osteo-chondral lesion; or
3. spread on the implant of the composition including the concentration of autologous mononuclear cells from medullary aspirate and support scaffold, the implant in turn is spread on the center of infection of the osteoarticular lesion; or
4. mixed in the composition according to point 1) and situated in the interface according to point 2); or
5. mixed in the composition according to point 1) and spread on the implant according to point 3); or
6. placed on the interface according to point 2) and spread on the implant according to point 3); or
7. contemporarily present in the composition according to point 1), placed on the interface according to point 2) and spread on the implant according to point 3).

The rationale of the method of treatment of articular osteocartilaginous injuries in a single operating session, includes as a further preferred form:
- the drawing of venous blood the day before the operation for the preparation of the platelet gel with automated method, such as Vivostat® with possible freezing of the obtained platelet gel (these phases in optional form);
- in the operating site a medullary aspirate from the posterior iliac crest is performed, it is concentrated in the operating room by an automated method of
- centrifugation, with the use for instance of the Harvest device to get a concentration of mononuclear cells;
- during the concentration of the cells an arthroscopy or arthrotomy is performed of the joint to be treated with relief and recovery of the osteochondral lesion;
- the medullary concentrate is used for preparing a composition, preferably in paste form, by mixing with the collagen of pig origin (such as Spongostan® Powder or surgiflo®);
- the composition or paste is subsequently inserted by arthroscopy or arthrotomy to fill the site of the lesion;
- finally, optionally, a layer of platelet gel is spread onto it or, alternatively, the platelet gel can be placed on the bed of the lesion or in the composition or paste when the prepared platelet gel is used in the optional phases.
- A further object of the present invention includes the process for the preparation of the composition, in any of the preferred forms of realization described above, said process includes:
- Preparation of a concentration of autologous mononuclear cells from medullary aspirate with an automated method of centrifugation in the operating room, such as the BMAC® Harvest method;
- Preparation of an composition including the concentrate of autologous mononuclear cells from medullary aspirate and support scaffold chosen by the group including lyophilized scaffold or pre-constituted scaffold in the form of membrane or tissue, said composition obtained for mixing said components together.
Optionally said process of preparation includes in the phase of preparation of the composition the mixing of platelet gel obtained by the automated procedure with venous blood, or having obtained the composition of autologous. Mononuclear cells and scaffold, the distribution around the said composition, in contact with it, of platelet gel, preferably in layer form, obtained with the automated procedure 5 from venous blood.

## Claims

1. Composition including a medullary concentrate supported by a scaffold chosen from the group consisting of lyophilized scaffold and pre-constituted scaffold in the form of membrane or tissue.

2. Composition according to claim 1, wherein the medullary concentrate is withdrawn from the posterior iliac crest.

3. Composition according to claim 1 or 2, in which the scaffold is constituted by natural polymers, or semi-synthetic polymers, or synthetic polymers.

4. Composition according to claim 3, in which the natural polymers are selected from the group consisting of: collagen, collagen and glycosaminoglycan co-precipitates, hyaluronic acid, cellulose, polysaccharides in the form of chitin gel, chitosan, pectin or pectic acid, agar, agarose, xanthan, gellan gum, alginic acid or alginates, polymannans or polyglycans, starch and natural rubbers, and demineralized bone matrix (DBM).

5. The composition according to claim 4, wherein the collagen is pig collagen

6. Composition according to claim 3, in which the semi-synthetic polymers are selected from the group consisting of: reticulated collagen with agents such as aldehydes or their precursors, dicarboxylic acids or their halides, diammine, derivates of cellulose, hyaluronic acid, chitin gel or chitosan, gellan gum, xanthan, pectin or pectic acid, polyglycans, polymannans, agar, agarose, natural rubber and glycosaminoglycan.

7. Composition according to claim 3, in which the synthetic polymers are selected from the group consisting of: polylactic acid, polyglycolic acid or their copolymers or derivates, polydioxane, polyphosphazenes, polysulfonic resins, polyurethanes and PTFE, hydroxyapatite in paste or tissue form.

8. Composition according to claim 1, also including a platelet gel.

9. Composition according to claim 1, also including pharmacologically or biologically active substances.

10. Composition according to claims 1-9, in which the composition is cryopreserved.

11. Composition according to claims 1-10 for use as a medicament.

12. Composition according to claims 1-10, for use in the treatment of articular osteocartilaginous injuries in arthroscopic surgery in a single operating session.

13. Composition according to claims 1-10, for use in the treatment of articular osteocartilaginous injuries in arthrotomic surgery in a single operating session.

14. Process for the preparation of a composition including a medullary concentrate supported by scaffold chosen from the group consisting of lyophilized scaffold and pre-constituted scaffold in the form of membrane or tissue of the composition, including the steps of:
preparing a concentrate of medullary aspirate by automated method of centrifugation in the operating room;
preparing a composition including the concentrate and a support scaffold chosen from the group consisting of lyophilized scaffold and pre-constituted scaffold in the form of membrane or tissue, said composition obtained for mixing said components together.

15. The process according to claim 14, wherein the composition further includes a platelet gel.

## Patentansprüche

1. Zusammensetzung, einschließend ein Markkonzentrat, getragen von einem Gerüst, ausgewählt aus der Gruppe, bestehend aus gefriergetrocknetem Gerüst und vorgebildetem Gerüst in der Form einer Membran oder eines Gewebes.

2. Zusammensetzung nach Anspruch 1, wobei das Markkonzentrat dem rückseitigen Beckenkamm entnommen wird.

3. Zusammensetzung nach Anspruch 1 oder 2, wobei das Gerüst aus natürlichen Polymeren oder halb synthetischen Polymeren oder synthetischen Polymeren besteht.

4. Zusammensetzung nach Anspruch 3, wobei die natürlichen Polymere ausgewählt sind aus der Gruppe, bestehend aus Kollagen, Kollagen- und Glykosaminoglykan-Copräzipitaten, Hyaluronsäure, Cellulose, Polysacchariden in Form von Chitingel, Chitosan, Pectin oder Pectinsäure, Agar, Agarose, Xanthan, Gellangummi, Alginsäure oder Alginaten, Polymannanen oder Polyglykanen, Stärke und Naturkautschuk sowie demineralisierter Knochenmatrix (DKM).

5. Zusammensetzung nach Anspruch 4, wobei das Kollagen Schweinekollagen ist.

6. Zusammensetzung nach Anspruch 3, wobei die halb synthetischen Polymere ausgewählt sind aus der Gruppe, bestehend aus vernetztem Kollagen mit Wirkstoffen wie Aldehyden oder deren Präkursoren, Dicarboxylsäuren oder deren Haliden, Diaminen, Cellulosederivaten, Hyaluronsäure, Chitingel oder Chitosan, Gellangummi, Xanthan, Pectin oder Pectinsäure, Polyglykane, Polymannane, Agar, Agarose, Naturkautschuk und Glykosaminogklykan.

7. Zusammensetzung nach Anspruch 3, wobei die synthetischen Polymere ausgewählt sind aus der Gruppe, bestehend aus Polymilchsäure, Polyglykolsäure oder deren Copolymeren oder Derivaten, Polydioxanen, Polyphosphazenen, Polysulfonharzen, Polyurethanen und PTFE, Hydroxyapatiten in Pasten- oder Gewebeform.

8. Zusammensetzung nach Anspruch 1, auch einschließend ein Plättchengel.

9. Zusammensetzung nach Anspruch 1, auch einschließend pharmakologisch oder biologisch aktive Substanzen.

10. Zusammensetzung nach Anspruch 1 bis 9, wobei die Zusammensetzung kryopräserviert ist.

11. Zusammensetzung nach Anspruch 1 bis 10 für den Gebrauch als Medikament.

12. Zusammensetzung nach Anspruch 1 bis 10 für den Gebrauch bei der Behandlung osteokartilaginärer Gelenkverletzungen in der arthroskopischen Chirurgie bei einzelnen Operationssitzungen.

13. Zusammensetzung nach Anspruch 1 bis 10 für den Gebrauch bei der Behandlung osteokartilaginärer Gelenkverletzungen in der arthrotomischen Chirurgie bei einzelnen Operationssitzungen.

14. Verfahren zur Vorbereitung einer Zusammensetzung, einschließend ein Markkonzentrat, getragen von einem Gerüst, ausgewählt aus der Gruppe, bestehend aus gefriergetrocknetem Gerüst und vorgebildetem Gerüst in der Form einer Membran oder eines Gewebes der Zusammensetzung, umfassend folgende Schritte:
Vorbereiten eines Markaspiratkonzentrats durch eine automatische Zentrifugation im Operationssaal;
Vorbereiten einer Zusammensetzung, einschließend ein Markkonzentrat und ein Tragegerüst, ausgewählt aus der Gruppe, bestehend aus gefriergetrocknetem Gerüst und vorgebildetem Gerüst in der Form einer Membran oder eines Gewebes, wobei diese Zusammensetzung dadurch erhalten wird, dass die Komponenten vermischt werden.

15. Verfahren nach Anspruch 14, wobei die Zusammensetzung zudem ein Plättchengel umfasst.

## Revendications

1. Composition incluant un concentré médullaire supporté par une matrice choisie à partir du groupe consistant en une matrice lyophilisée et une matrice préconstituée sous forme de membrane ou de tissu.

2. Composition selon la revendication 1, dans laquelle le concentré médullaire est prélevé de la crête iliaque postérieure.

3. Composition selon les revendications 1 ou 2, dans laquelle la matrice est constituée de polymères naturels ou de polymères semi-synthétiques ou de polymères synthétiques.

4. Composition selon la revendication 3, dans laquelle les polymères naturels sont sélectionnés à partir du groupe composé des éléments suivants : collagène, et coprécipités de collagène et de glycosaminoglycane, acide hyaluronique, cellulose, polysaccharides en gel de chitine, chitosane, pectine ou d'acide pectique, d'agar, d'agarose, de xanthane, de gomme gellane, d'acide alginique ou d'alginates, de polymannans ou polyglycanes, d'amidon ou de caoutchoucs naturels et de matrice osseuse déminéralisée (DBM).

5. Composition selon la revendication 4, dans laquelle le collagène est un collagène porcin.

6. Composition selon la revendication 3, dans laquelle les polymères semi-synthétiques sont sélectionnés à partir du groupe composé des éléments suivants :
collagène réticulé avec des agents tels que des aldéhydes ou leurs précurseurs, des acides dicarboxyliques ou leurs halogénures, diammine, dérivés de cellulose, acide hyaluronique, gel de chitine ou chitosane, gomme gellane, xanthane, pectine ou d'acide pectique, polyglycanes, polymannans, d'agar, d'agarose, caoutchouc naturel et glycosaminoglycanes.

7. Composition selon la revendication 3, dans laquelle les polymères synthétiques sont sélectionnés dans le groupe constitué des éléments suivants : acide polylactique, acide polyglycolique ou leurs copolymères ou dérivés, polydioxanone, polyphosphazènes, résines de polysulfone, polyuréthannes et PTFE, hydroxyapatite sous forme de pâte ou de tissu.

8. Composition selon la revendication 1, incluant aussi un gel plaquettaire.

9. Composition selon la revendication 1, incluant aussi des substances actives pharmacologiquement ou biologiquement.

10. Composition selon les revendications 1 à 9, dans laquelle la composition est cryoconservée.

11. Composition selon les revendications 1 à 10 pour son utilisation en tant que médicament.

12. Composition selon les revendications 1 à 10, pour son utilisation dans le traitement des blessures articulaires ostéo-cartilagineuses en chirurgie arthroscopique au cours d'une unique opération chirurgicale.

13. Composition selon les revendications 1 à 10, pour son utilisation dans le traitement des blessures articulaires ostéo-cartilagineuses en chirurgie arthrotomique au cours d'une unique opération chirurgicale.

14. Procédé de préparation d'une composition incluant un concentré médullaire supporté par une matrice choisie à partir du groupe consistant en une matrice lyophilisée et une matrice préconstituée sous forme de membrane ou de tissu de la composition, incluant les étapes de :
Préparer un concentré d'aspirat médullaire par une méthode automatisée de centrifugation dans la salle d'opération ;
Préparer une composition incluant le concentré et une matrice de support choisie à partie du groupe consistant en une matrice lyophilisée et une matrice préconstituée sous forme de membrane ou de tissu, ladite composition est obtenue pour mélanger lesdits composants entre eux.

15. Procédé selon la revendication 14, dans lequel la composition inclut de plus un gel plaquettaire.
